**Europäisches Patentamt**

**European Patent Office**

**Office européen des brevets**

(19)

(11) Publication number : **0 503 886 A1**

(12) # EUROPEAN PATENT APPLICATION

(21) Application number : **92302012.7**

(51) Int. Cl.⁵ : **C12Q 1/32**

(22) Date of filing : **10.03.92**

(30) Priority : **11.03.91 JP 69401/91**

(43) Date of publication of application :
**16.09.92 Bulletin 92/38**

(84) Designated Contracting States :
**DE FR GB**

(71) Applicant : **TAKARA SHUZO CO. LTD.**
**609 Takenakacho**
**Fushimi-ku Kyoto (JP)**

(72) Inventor : **Yamauchi, Masayoshi**
**1-8-10-426, Misyuku**
**Setagaya-ku, Tokyo-to (JP)**
Inventor : **Ohata, Mitsuru**
**5-18-11, Kamiikedai**
**Oota-ku, Tokyo-to (JP)**
Inventor : **Kimura, Kazuo**
**1330-16, Okamiya**
**Numazu-shi, Shizuoka-ken (JP)**
Inventor : **Katayama, Kaoru**
**1496-6-409, Kusatsu-cho**
**Kusatsu-shi, Shiga-ken (JP)**

(74) Representative : **Marlow, Nicholas Simon**
**Reddie & Grose 16, Theobalds Road**
**London WC1X 8PL (GB)**

(54) **Method for detecting liver diseases.**

(57)    This invention provides a simple method for definitely detecting the cause of liver cirrhosis, which comprises measuring the amount of free L-fucose in specimens taken from urine and comparing the measured value with the control value, thereby detecting alcoholic liver cirrhosis.

   As the control value, may be used the normal value and the value for viral liver cirrhosis. Compared with each of these values, specimens taken from urine of patients with alcoholic liver cirrhosis contain a larger amount of L-fucose.

EP 0 503 886 A1

This invention relates to a method for detecting liver diseases. More particularly, it relates to a method for definitely detecting alcoholic liver cirrhosis.

[ Prior Art ]

Various forms of glycoconjugates, such as glycoproteins and glycolipids, are present in the living bodies of higher animals. Many of these glycoconjugates contain L-fucose as a constituent carbohydrate in the sugar chain. As typical examples of the glycoconjugate containing L-fucose in its sugar chain, there may be mentioned, among others, the mucous glycoprotein that serves to protect the gastric mucous, the mucous glycoprotein secreted from the submaxillary glands, the $\alpha_1$-acid glycoprotein and $\beta_2$-glycoprotein synthesized and secreted by the liver, the glycoprotein on the surface of erythrocyte membrane and $\gamma$-globulin formed in the bone marrow, and the glycoprotein and glycolipid present on the surface of cell membrane. As mentioned above, a large amount of glycoconjugates containing L-fucose is present in living bodies. On the other hand, it is known that changes in the sugar chain structure in the glycoconjugates containing L-fucose or changes in the amount of the glycoconjugates in the body fluid are observed in specific diseases; for example, the reduced secretion of the mucous glycoprotein in the case of gastric ulcer and cancer, the reduced secretion of glycoproteins, such as $\alpha_1$-acid glycoprotein, in the case of liver cirrhosis and hepatoma, and the changes in the sugar chain structure of glycoproteins and glycolipids on the cell membrane surface resulting from carcinogenesis of the cells have been reported.

It is also known that specimens taken from patients with specific diseases, such as gastric ulcer, gastric cancer, liver cirrhosis, hepatoma and other cancers, contain a layer amout of free L-fucose than those taken from healthy subject (U.S. Patent No.4927767).

[ Problem to be Solved by the Invention ]

Liver cirrhosis, which is associated with metabolic abnormality of L-fucose, is a serieous disease showing a progressive course, and its cause may be classified into two types: viral and alcoholic cirrhosis. The therapeutics to be applied to a case diagnosed as liver cirrhosis should be greatly different according to its cause, and there has been a demand for establishing an early diagnosis of its cause.

The object of this invention is to provide a simple method for definitely diagnosing the cause of liver cirrhosis.

[ Means to Solve the Problem ]

In summary, this invention relates to a method for detecting alcohlic liver cirrhosis, which is characterized in that the amount of free L-fucose in specimens taken from patients is measured and the measured value is compared with the control value.

As the control value, may be used the normal value and the valve for vital liver cirrhosis. Compared with each of these values, specimens taken from urine of patients with alcoholic liver cirrhoris contain a larger amount of L-fucose.

The specimens used in this invention for detecting alcoholic liver cirrhosis are taken from human urine; the urine itself is usually employed as the specimen, and a specimen may also be prepared by infiltration of the urine into a definite amount of an absorbent, such as filter paper, followed by extraction. Such specimens can be taken with no need for the blood-gathering operation, and are therefore suitable also in the group checkup which requires treatment of a large amount of specimens, in terms of simple operation, bulk handling and low cost.

Quantitative analysis of free L-fucose contained in uric specimens may be effected by the enzyme reaction using L-fucose dehydrogenase as described in U.S. Patent No.4927767 and Clinical Chemistry, 36, 474-476 ( 1990 ), or by a method using HPLC, for example, according to the fluorescent labelling method for reducing sugars described in U.S. Patent No.4975533, in which the free L-fucose contained in the specimens is fluorescently labelled by the use of 2-aminopyridine, and the labelled compound is detected by HPLC.

The above-mentioned method described in Clinical Chemistry is to allow $NAD^+$ and fucose dehydrogenase to act upon the free L-fucose contained in urine, and to determine the increased amount of NADH thus formed by measuring the change in absorbance at a wavelength of 340 nm, thus effecting the quantitative analysis of the free L-fucose; this is a simple and rapid process comprising the reactions of a uric specimen and a standard solution of fucose each with $NAD^+$ and fucose dehydrogenase, and analysis of the changes in absorbance by the use of an automatic analyzer. In addition, the amount of creatinine ( Cr ) contained in the uric specimen is also measured simultaneously to perform correction of the urine volume by the Jaff'e's reaction [ Clinical Chemistry, 17, 696-700 ( 1971 )], and the amount of free L-fucose in the uric specimen is expressed by μmol/g·Cr.

When the method described above is adopted to measure the amount of free L-fucose in urine for 58 healthy subjects, 17 patients with viral liver cirrhosis and 7 patients with alcoholic liver cirrhosis, the μmol/g.Cr value is 144±30 ( average±standard deviation ), 268±41 and 350±84 for the healthy subjects, for the viral cirrhosis patients and for the alcoholic cirrhosis patients, respectively, indicating that liver cirrhosis patients show significantly higher values than healthy subjects and that there is marked difference in the L-fucose content between the alcoholic cirrhosis patients and the viral cirrhosis patients, thus enabling definite diagnosis of alcoholic liver cirrhosis.

[ Examples ]

The following Example will further illustrate the invention but is not intended to limit its scope.

Example 1

The amount of free L-fucose contained in urine was measured for 58 healty subjects, 17 patients with viral liver cirrhosis and 7 patients with alcoholic liver cirrhosis by using automatic analyzer ( product of Hitachi, Ltd.; Modle 7150 ) as described below.

A mixture of 10 μl urine and 300 μl Reagent A ( containing 100 mM potassium phosphate buffer ( pH 8.0 ), 0.1% Brij-35 surfactant and 0.05% $NaN_3$ ) was held at 37°C for five minutes, and its absorbance at 340 nm was measured ( absorbance: $A_1$ ). 100 μl of Reagent B ( a mixture of 1 ml Reagent A, 60 units L-fucose dehydrogenase ( Takara Shuzo Co., Ltd. ) and 5 μ mol $NAD^+$) was then added, the resulting mixture was held at 37°C for five minutes, and its absorbance at 340 nm was measured ( absorbance: $A_2$ ).

The increase in absorbance resulting from the presence of L-fucose in urine ( $A_3$ ) was obtained by calculation according to the folowing equation:

$$A_3 = A_2 - (300 + 10)A_1/(10 + 300 + 100)$$

The standard curve for L-fucose was obtained by an experiment in much the same reaction system as above, except that water and 1 mM L-fucose were used in place of the urine, and the L-fucose content of uric specimens was obtained from absorbance $A_3$ by using the standard curve.

The results obtained are shown in Table 1 below.

Table 1

| Specimens | | Free L-fucose content of urine (μmol/g·Cr) |
|---|---|---|
| Taken from | Number of subjects | Average±Standard deviation |
| Healthy subjects | 58 | 144±30 |
| Viral liver cirrhosis patients | 17 | 268±41 |
| Alcoholic liver cirrhosis patients | 7 | 350±84 |

As is apparent from the table, it was confirmed that alcoholic liver cirrhosis patients show a larger amount of free L-fucose contained in urine than viral liver cirrhosis patients.

**Claims**

1. A method for detecting alcoholic liver cirrhosis, which is characterized in that the amount of free L-fucose contained in specimens taken urine is measured and the measured value is compared with the control value.

European Patent Office

**EUROPEAN SEARCH REPORT**

Application Number

EP    92 30 2012

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| D,X | DE-A-3 838 718 (TAKARA SHUZO CO., LTD.) <br> * the whole document * | 1 | C12Q1/32 |
| | ----- | | |
| | | | **TECHNICAL FIELDS SEARCHED (Int. Cl.5 )** |
| | | | C12Q |

The present search report has been drawn up for all claims

| Place of search | Date of completion of the search | Examiner |
|---|---|---|
| BERLIN | 19 JUNE 1992 | DE KOK A.J. |

EPO FORM 1503 03.82 (P0401)

4